Europäisches Patentamt

European Patent Office

Office européen des brevets

(11) Publication number: **0 209 607**
**B1**

(12) **EUROPEAN PATENT SPECIFICATION**

(45) Date of publication of patent specification: **20.12.89**

(21) Application number: **85106727.2**

(22) Date of filing: **31.05.85**

(51) Int. Cl.⁴: **A 61 M 1/14, B 67 D 1/04**

(54) **Hemodialysis liquid feed system.**

(43) Date of publication of application:
**28.01.87 Bulletin 87/05**

(45) Publication of the grant of the patent:
**20.12.89 Bulletin 89/51**

(84) Designated Contracting States:
**AT BE CH DE FR GB IT LI NL SE**

(56) References cited:
**FR-A-1 435 806**
**FR-A-2 504 817**
**GB-A-2 069 855**

(73) Proprietor: **Dietl, Hans, Dr.**
**Eichendorffstrasse 33**
**D-8202 Bad Aibling (DE)**

(72) Inventor: **Dietl, Hans, Dr.**
**Eichendorffstrasse 33**
**D-8202 Bad Aibling (DE)**

(74) Representative: **Patentanwälte Müller-Boré,**
**Deufel, Schön, Hertel, Lewald, Otto**
**Postfach 26 02 47 Isartorplatz 6**
**D-8000 München 26 (DE)**

Courier Press, Leamington Spa, England.

## Description

This invention relates to a hemodialysis liquid feed system for feeding hemodialysis liquid to one or more hemodialysis machines, which comprises at least one principal container for containing the hemodialysis liquid or a liquid component thereof and to a method of producing a stable aqueous sodium bicarbonate solution and such a stable aqueous sodium bicarbonate solution.

More particularly this invention relates to a hemodialysis liquid feed system providing not only means for driving hemodialysis liquid or liquid components or liquid concentrates thereof from a container containing such liquids, but also means for securing the stability of otherwise unstable hemodialysis liquid components, in particular sodium bicarbonate solution or concentrate.

Present hemodialysis liquid feed systems, i.e. for feeding a concentrate from a container to a proportioning system where the concentrate is diluted to the correct dilution for use in a hemodialysis procedure, normally involve the use of pumps although gravity feed systems from central delivery systems have been considered. It has now been found, in accordance with the invention, that gas pressure applied in containers, for example containing a hemodialysis liquid concentrate or a liquid component thereof provides a very convenient and useful driving force which is readily available. This aspect, coupled with the fact that the gas employed in accordance with the invention is carbon dioxide, provides means for stabilizing sodium bicarbonate concentrates or the sodium bicarbonate dialysis liquid component of the dialysis procedure known as sodium bicarbonate dialysis.

Sodium bicarbonate dialysis has over the past years become recognised to be preferable to acetate dialysis. Acetate comprised in acetate dialysis liquid in acetate dialysis is transported into the blood stream of a patient undergoing treatment across the membrane of a membrane exchange device. The acetate is then metabolised by the liver to bicarbonte and in this fashion depleted bicarbonate buffer reserves can be restored. At least the initial stages of acetate dialysis can be uncomfortable for some patients and certain patients do not appear to tolerate acetate as well as others. Bicarbonate dialysis on the other hand provides an immediate availability of bicarbonate in the blood stream.

One factor which has had a limiting effect on change from acetate dialysis to bicarbonate dialysis is the poor stability of bicarbonate solutions. The sodium bicarbonate forms sodium carbonate and loses carbon dioxide to the atmosphere. This leads to a lowering of bicarbonate content in the solution and an increase in the pH of the solution as a result of the presence of sodium carbonate formed.

A method and an apparatus for preparing fluids of accurately proportioned components is disclosed in GB—A—2 069 855. In this known apparatus carbon dioxide is dissolved in water in a mixer. After this the water containing the dissolved carbon dioxide is heated and the gas is removed from the fluid by a vent device and the fluid is used as dilute fluid which is mixed with a concentrate fluid component and a sodium bicarbonate solution.

In accordance with the invention, it has been found that sodium bicarbonate solutions, or concentrates, useful in bicarbonate dialysis, can be stabilized by introducing a freshly prepared aqueous bicarbonate solution into an air-tight container, displacing air above the level of the solution with carbon dioxide, and introducing carbon dioxide under pressure into the container so that pressure in the container is in excess of atmospheric pressure. The pressure in the container is preferably at least 1.2 atmospheres and up to 2.5 atmospheres, a pressure of about 1.8 atmospheres being preferred.

The same considerations as above apply to hemodialysis liquid feed systems where hemodialysis liquid or hemodialysis liquid components, such as the above-mentioned bicarbonate component, are preferably available in storage for ready use. Thus, a hemodialysis liquid feed system in accordance with the invention comprises at least one principal container for containing the hemodialysis liquid or a liquid component thereof, and is characterized in that the principal container contains a sodium bicarbonate hemodialysis liquid component and in that the system comprises connection means for connecting said principal container to a source of carbon dioxide under super-atmospheric pressure, pressure regulator means operative between the source of carbon dioxide and the principal container to regulate the pressure value in the principal container and maintain this at a value in excess of atmospheric pressure, and conduit means leading from the principal container for connection to the one or more hemodialysis machines. The gas pressure in the principal container most preferably provides a force on liquid in the container which is adequate to drive the liquid to the hemodialysis machine.

The acid hemodialysis liquid component will for example comprise such components as magnesium, calcium, chloride, hydrogen ions, and an amount of acetate which assists in maintaining sterility of the solution. The bicarbonate component needs to be separated from the acid component since precipitation of calcium and magnesium would otherwise take place.

So that a more convenient means is available for sensing depletion of a principal container, a subsidiary container may be provided downstream of the principal container, i.e. intermediate the principal container and the hemodialysis machine. The principal container, or if a subsidiary container is provided may comprise level sensor means and an electrovalve may be provided in the conduit and arranged to close automatically when the level of liquid in the container falls below a predetermined level.

A conductivity probe may furthermore be provided in the conduit means for measuring the conductivity of liquid flowing to the hemodialysis machine. The electrovalve mentioned above may similarly be arranged to close automatically if the conductivity of the liquid is not within predetermined limit values.

The invention will now be described with reference to the accompanying drawings showing an exemplary hemodialysis liquid feed system of the invention. In the drawings:

Figure 1 is a schematic diagram of a hemodialysis liquid feed system; and

Figure 2 is a side view of principal containers arranged in tandem fashion, which containers may also find application for transport of hemodialysis liquids.

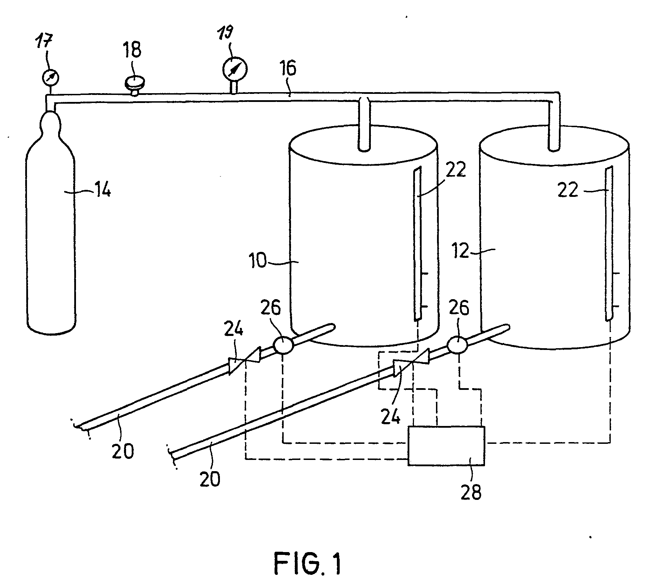

Referring to Figure 1, principal containers 10 and 12 are provided for containing an acid component and a bicarbonate component. A source of carbon dioxide 14 is connected to each of the containers 10 and 12 via gas leads 16. Pressure regulator means 18 is operative between the source of carbon dioxide 14 and the principal container 10 and 12, which is employed to regulate the pressure in the containers and maintain this at a value in excess of atmospheric pressure. Reference numerals 17 and 19 respectively refer to manometers for measuring the gas pressure in carbon dioxide source 14 and in gas leads 16.

Conduit means 20 lead from the principal containers 10 and 12 for connection to one or more hemodialysis machines (not shown). Level sensor means 22 are provided in each of the containers for sensing the level of dialysis liquid or component in the containers. Electrovalves 24 are arranged to close automatically, via electronic control 28 when the level of liquid falls below a predetermined level. Conductivity probes 26 are provided to measure the conductivity of solutions passing through the conduits 20 and the electrovalves 24 are similarly arranged to close automatically via electronic control 28 if the conductivity of the solution deviates outside of predetermined limit values.

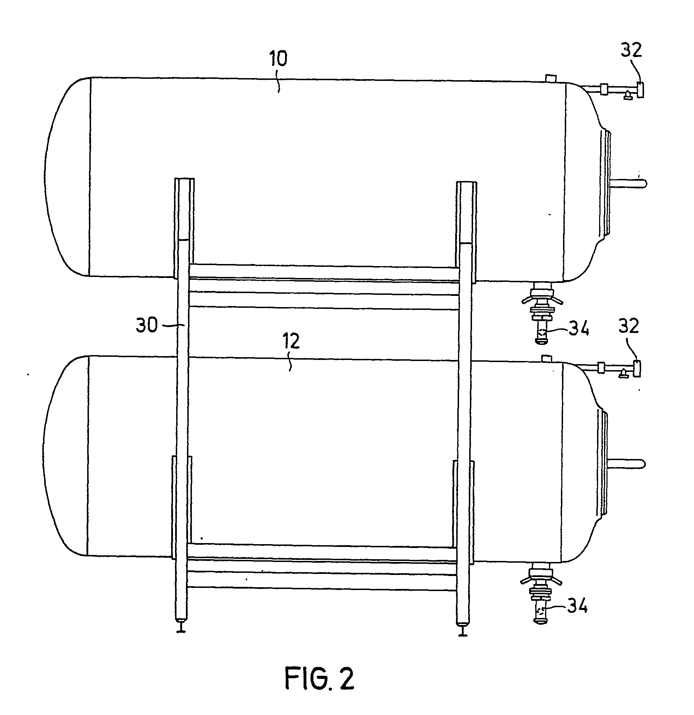

In Figure 2, the principal containers 10, 12 are mounted in tandem fashion on a stand 30. Connection means 32, 34 are provided for respective connection to the carbon dioxide source 14 and to conduit means 20 shown in Figure 1.

## Claims

1. A hemodialysis liquid feed system for feeding hemodialysis liquid to one or more hemodialysis machines, which comprises at least one principal container (10, 12) for containing the hemodialysis liquid or a liquid component thereof, characterized in that the principal container contains a sodium bicarbonate hemodialysis liquid component and in that the system comprises connection means (16) for connecting said principal container to a source of carbon dioxide (14) under super-atmospheric pressure, pressure regulator means (18) operative between the source of carbon dioxide and the principal container to regulate the pressure value in the principal container and maintain this at a value in excess of atmospheric pressure, and conduit means (20) leading from the principal container for connection to the one or more hemodialysis machines.

2. A hemodialysis liquid feed system according to claim 1, in which the pressure value in the principal container provides a force on hemodialysis liquid or a liquid component thereof in the principal container, which force is adequate to drive the hemodialysis liquid or liquid component from the principal container to the hemodialysis machine.

3. A hemodialysis liquid feed system according to claim 1 or claim 2, in which two principal containers (10, 12) are provided, one for containing one hemodialysis liquid component and the other for containing another hemodialysis liquid component.

4. A hemodialysis liquid feed system according to any one of the preceding claims, in which a subsidiary container is provided in the conduit means, intermediate the principal container and the one or more hemodialysis machines.

5. A hemodialysis liquid feed system according to any one of the preceding claims, in which level sensor means is provided for sensing the level of hemodialysis liquid or hemodialysis liquid component in the principal container or subsidiary container.

6. A hemodialysis liquid feed system according to claim 5, in which an electrovalve is provided in the conduit means which is arranged to automatically close the conduit means in response to a signal from the level sensor when the level of liquid in the container falls below a predetermined level.

7. A hemodialysis liquid feed system according to any one of the preceding claims, in which a conductivity probe is provided in the conduit means for measuring the conductivity of the hemodialysis liquid or hemodialysis liquid component flowing in the conduit means.

8. A hemodialysis liquid feed system according to claim 7, in which an electrovalve is provided in the conduit means which is arranged to automatically close the conduit means in response to a signal from the conductivity probe when the conductivity of the hemodialysis liquid or hemodialysis liquid component deviates outside of predetermined limit values.

9. A method of producing a stable aqueous sodium bicarbonate solution, which comprises the steps of introducing a freshly prepared aqueous bicarbonate solution into an air-tight container, displacing air above the level of the solution with carbon dioxide, and introducing carbon dioxide under pressure into the container so that pressure in the container is in excess of atmospheric pressure.

10. A stable aqueous sodium bicarbonate solution in which the space above the level of a freshly prepared bicarbonate solution contained in an air-tight container is filled with carbon dioxide and in which the carbon dioxide is under super-atmospheric pressure.

## Patentansprüche

1. Förderungssystem für Hämodialyseflüssigkeit zum Zuführen von Hämodialyseflüssigkeit zu einer oder mehreren Hämodialysemaschinen, welches wenigstens einen Hauptbehälter (10, 12) zur Aufnahme der Hämodialyseflüssigkeit oder einer Flüssigkeitskomponente von dieser aufweist, dadurch gekennzeichnet, daß der Hauptbehälter eine Natriumbikarbonat-Hämodialyseflüssigkeitskomponente enthält und daß das System eine Verbindungseinrichtung (16) zum Verbinden des Hauptbehälters mit einer Quelle von unter Überatmosphärendruck stehendem Kohlendioxyd (14), eine Druckregeleinrichtung (18), die zwischen der Kohlendioxydquelle und dem Hauptbehälter betätigbar ist, um den Druckwert in dem Hauptbehälter zu regeln und diesen auf einem Wert über Atmosphärendruck zu halten, und eine Leitungseinrichtung (20) aufweist, das von dem Hauptbehälter zur Verbindung mit der einen oder den mehreren Hämodialysemaschinen geführt ist.

2. Förderungssystem für Hämodialyseflüssigkeit nach Anspruch 1, wobei der Druckwert in dem Hauptbehälter eine Kraft auf die Hämodialyseflüssigkeit oder eine Flüssigkeitskomponente von dieser in dem Hauptbehälter ausübt, welche geeignet ist, die Hämodialyseflüssigkeit oder die Flüssigkeitskomponente von dem Hauptbehälter zu der Hämodialysemaschine zu fördern.

3. Förderungssystem für Hämodialyseflüssigkeit nach Anspruch 1 oder 2, wobei zwei Hauptbehälter (10, 12) vorgehen sind und wobei einer zur Aufnahme einer Hämodialyseflüssigkeitskomponente und der andere zur Aufnahme einer anderen Hämodialyseflüssigkeitskomponente vorgesehen ist.

4. Förderungssystem für Hämodialyseflüssigkeit nach einem der vorhergehenden Ansprüche, wobei ein Nebenbehälter in der Leitungseinrichtung zwischen dem Hauptbehälter und der einen oder den mehreren Hämodialysemaschinen vorgesehen ist.

5. Förderungssystem für Hämodialyseflüssigkeit nach einem der vorhergehenden Ansprüche, wobei eine Pegelsensoreinrichtung vorgesehen ist zur Erfassung des Pegels der Hämodialyseflüssigkeit oder der Hämodialyseflüssigkeitskomponente in dem Hauptbehälter oder dem Nebenbehälter.

6. Förderungssystem für Hämodialyseflüssigkeit nach Anspruch 5, wobei ein Elektroventil in der Leitungseinrichtung vorgesehen ist, welches angeordnet ist, um automatisch die Leitungseinrichtung ansprechend auf ein Signal von dem Pegelsensor zu schließen, wenn der Flüssigkeitspegel in dem Behälter unter einen vorbestimmten Pegel absinkt.

7. Förderungssystem für Hämodialyseflüssigkeit nach einem der vorhergehenden Ansprüche, wobei eine Leitfähigkeitssonde in der Leitungseinrichtung angeordnet ist zum Messen der Leitfähigkeit der Hämodialyseflüssigkeit oder der Hämodialyseflüssigkeitskomponente, die in der Leitungseinrichtung fließt.

8. Förderungssystem für Hämodialyseflüssigkeit nach Anspruch 7, wobei ein Elektroventil in der Leitungseinrichtung vorgesehen ist, welches angeordnet ist, um automatisch die Leitungseinrichtung ansprechend auf ein Signal von der Leitfähigkeitssonde zu schließen, wenn die Leitfähigkeit der Hämodialyseflüssigkeit oder der Hämodialyseflüssigkeitskomponente von den vorbestimmten Grenzwerten abweicht.

9. Verfahren zur Herstellung einer stabilen wässrigen Natriumbikarbonatlösung, welches die Schritte der Einleitung einer frisch vorbereiteten wässrigen Bikarbonatlösung in einen luftdichten Behälter, des Austauschs von Luft über dem Spiegel der Lösung mit Kohlendioxyd und des Einleitens von Kohlendioxyd unter Druck in den Behälter umfaßt, so daß der Druck in dem Behälter über Atmosphärendruck liegt.

10. Stabile wässrige Natriumbikarbonatlösung, wobei der Raum über dem Spiegel einer frisch vorbereiteten Bikarbonatlösung, die in einem luftdichten Behälter enthalten ist, mit Kohlendioxyd gefüllt wird und wobei das Kohlendioxyd unter einem Druck über dem Atmosphärendruck steht.

## Revendications

1. Système d'alimentation en liquide d'hémodialyse pour alimenter en liquide d'hémodialyse une ou plusieurs machines d'hémodialyse, lequel comprend au moins un réservoir principal (10, 12) pour contenir le liquide d'hémodialyse ou un composant liquide de celui-ci, caractérisé en ce que le réservoir principal contient un composant liquide d'hémodialyse à base de bicarbonate de sodium et en ce que le système comporte des moyens de raccordement (16) pour raccorder ledit réservoir principal à une source de dioxyde de carbone (14) en hyperpression, un moyen de régulation de pression (18) disposé fonctionnellement entre la source de dioxyde de carbone et le réservoir principal pour réguler la valeur de la pression dans le réservoir principal et la maintenir à une valeur supérieure à la pression atmosphérique, et des moyens de canalisation (20) partant du réservoir principal pour être raccordés à l'une ou plusieurs machines d'hémodialyse.

2. Système d'alimentation en liquide d'hémodialyse selon la revendication 1, dans lequel la valeur de la pression dans le réservoir principal fournit une force s'exerçant sur le liquide d'hémodialyse ou un composant liquide de celui-ci dans le réservoir principal, laquelle force est suffisante pour entraîner le liquide d'hémodialyse ou le composant liquide du réservoir principal à la machine d'hémodialyse.

3. Système d'alimentation en liquide d'hémodialyse selon la revendication 1 ou la revendication 2, dans lequel sont disposés deux réservoirs principaux (10, 12), l'un pour contenir un composant liquide d'hémodialyse et l'autre pour contenir un autre composant liquide d'hémodialyse.

4. Système d'alimentation en liquide d'hémodialyse selon l'une quelconque des revendications précédentes, dans lequel un réservoir auxiliaire est disposé dans les moyens de canalisation entre le réservoir principal et l'une ou plusieurs machines d'hémodialyse.

5. Système d'alimentation en liquide d'hémodialyse selon l'une quelconque des revendications précédentes, dans lequel un moyen de détection de niveau est aménagé pour détecter le niveau du liquide d'hémodialyse ou du composant liquide d'hémodialyse dans le réservoir principal ou le réservoir auxiliaire.

6. Système d'alimentation en liquide d'hémodialyse selon la revendication 5, dans lequel une électrovanne est disposée dans les moyens de canalisation et aménagée pour fermer automatiquement les moyens de canalisation en réponse à un signal du détecteur de niveau lorsque le niveau du liquide dans le réservoir tombe en dessous d'un niveau prédéterminé.

7. Système d'alimentation en liquide d'hémodialyse selon l'une quelconque des revendications précédentes, dans lequel la sonde de conductibilité est disposée dans les moyens de canalisation pour mesurer la conductivité du liquide d'hémodialyse ou du composant liquide d'hémodialyse s'écoulant dans les moyens de canalisation.

8. Système d'alimentation en liquide d'hémodialyse selon la revendication 7, dans lequel une électrovanne est disposée dans les moyens de canalisation et aménagée pour fermer automatiquement les moyens de canalisation en réponse à un signal de la sonde de conductibilité lorsque la conductivité du liquide d'hémodialyse ou du composant liquide d'hémodialyse s'écarte à l'extérieur de valeurs limites prédéterminées.

9. Procédé d'obtention d'une solution aqueuse stable de bicarbonate de sodium, qui comprend les étapes où l'on introduit une solution aqueuse de bicarbonate fraîchement préparée, on déplace l'air au-dessus du niveau de la solution avec du dioxyde de carbone, et on introduit du dioxyde de carbone sous pression dans le réservoir de façon à ce que la pression dans le réservoir soit supérieure à la pression atmosphérique.

10. Solution aqueuse stable de bicarbonate de sodium dans laquelle l'espace au-dessus du niveau d'une solution de bicarbonate fraîchement préparée et contenue dans un réservoir hermétique, est rempli avec du dioxyde de carbone et dans laquelle le dioxyde de carbone est en hyperpression.

FIG. 1

FIG. 2